# EUROPEAN PATENT SPECIFICATION

(11) **EP 4 322 924 B1**
(45) Date of publication and mention of the grant of the patent: **30.04.2025**
(21) Application number: 22722561.2
(22) Date of filing: 15.04.2022
(51) Int. Cl.: A61K 9/50, A61K 9/107

(54) **SOLID MICROCAPSULE HAVING SELF-EMULSIFYING CAPACITY**
FESTE MIKROKAPSEL MIT SELBSTEMULGIERENDER FÄHIGKEIT
MICROENCAPSULE SOLIDE À CAPACITÉ AUTO-ÉMULSIFIANTE

(30) Priority: 16.04.2021 IT 202100009650
(43) Date of publication of application: 21.02.2024
(73) Proprietor: Salix S.r.l., 36030 Monte di Malo (IT)
(72) Inventor: BUSON, Cristina, 36070 Brogliano (IT); BAGGIO, Roberto, 31011 Asolo (IT)
(74) Representative: Cattaneo, Elisabetta
(86) International application number: PCT/IB2022/053565
(87) International publication number: WO 2022/219599

(56) References cited:
- ES-A1- 2 168 997
- "Handbook of Pharmaceutical Excipients ED - AMERICAN PHARMACEUTICAL ASSOCIATION & THE PHARMACEUTICAL SOCIETY OF GREAT BRITAIN", 1 January 1986, HANDBOOK OF PHARMACEUTICAL EXCIPIENTS, WASHINGTON, AM. PHARMACEUTICAL ASSOC, US, PAGE(S) 314 - 320, XP002156607
- ATTAMA A A ET AL: "Further characterization of theobroma oil-beeswax admixtures as lipid matrices for improved drug delivery systems", EUROPEAN JOURNAL OF PHARMACEUTICS AND BIOPHARMACEUTICS, ELSEVIER SCIENCE PUBLISHERS B.V., AMSTERDAM, NL, vol. 64, no. 3, 1 November 2006 (2006-11-01), pages 294 - 306, XP027997931, ISSN: 0939-6411, [retrieved on 20061101], DOI: 10.1016/J.EJPB.2006.06.010
- AMEKYEH HILDA ET AL: "Lyophilized Drug-Loaded Solid Lipid Nanoparticles Formulated with Beeswax and Theobroma Oil", MOLECULES, vol. 26, no. 4, 9 February 2021 (2021-02-09), pages 908, XP055876580, DOI: 10.3390/molecules26040908

## Description

### FIELD OF THE INVENTION

The invention relates to a solid particle having self-emulsifying capacity and usable for the microencapsulation of active ingredients.

### STATE OF THE ART

Self-emulsifying systems used in pharmaceutical applications to increase the solubility of insoluble active ingredients which, due to this characteristic, are poorly bioavailable (Lipinski C.A., Drug-like properties and the causes of poor solubility and poor permeability, J. Pharmacol. Toxicol. Methods, 44:235-249, 2000) are extensively studied in the literature. Lipid matrices for improved drug delivery have also been investigated (Attama A.A., Further characterization of theobroma oil-beeswax admixtures as lipid matrices for improved drug delivery systems, Eur. J. Pharm. and Biopharm, 64:294-306, 2006).

The systems studied are defined as "Self-Emulsifying Drug Delivery Systems" (SEDDS) and are characterized by the ability to generate sufficiently stable emulsions in water under mild stirring conditions. This differentiates them from the classic emulsions in which the homogeneous dispersion is obtained through vigorous stirring. This feature is very important because inside the body, an emulsion can only be formed through the mild mechanical movements applied by the intestinal motion.

Self-emulsifying systems involve the use of oily substances, emulsifying substances, and co-emulsifying substances.

The components of these systems are known to have the following reasons for use:
- oily substances are used to solubilize water insoluble and generally lipophilic active ingredients. This allows the active ingredients to be in a liquid vehicle ready to get emulsified;
- emulsifiers, or surfactants, are ingredients that drive the formation of the emulsion and make the dispersed system sufficiently stable, preventing coalescence of the oily portion immiscible with water;
- co-emulsifiers favor the formation of the emulsion, reduce the energy needed to form the emulsion and favor the its stabilization (Kang BK, Lee JS, Chon SK, Jeong SY, Yuk SH, Khang G, Lee HB, Cho SH. Development of self-microemulsifying drug delivery systems (SMEDDS) for oral bioavailability enhancement of simvastatin in beagle dogs. Int J Pharm. 2004 Apr 15;274(1-2):65-73. doi: 10.1016/j.ijpharm.2003.12.028. PMID: 15072783).

The active ingredient included in these systems is generally characterized by poor solubility in water and poor bioavailability in the body following intake. Making an emulsion of the active dissolved in the oily phase involves the solubilization of the active in water, moreover it generally involves an increase in the bioavailability of the active thus delivered (Gursoy, R.N. and Benita, S. (2004) Self-emulsifying drug delivery systems (SEDDS) for improved oral delivery of lipophilic drugs. Biomed. Pharmacother. 58, 173-182).

In the paper by Mauro Serratoni et al (Mauro Serratoni, Michael Newton, Steven Booth, Ashley Clarke, Controlled drug release from pellets containing water-insoluble drugs dissolved in a self-emulsifying system, European Journal of Pharmaceutics and Biopharmaceutics, Volume 65, Issue 1, 2007, Pages 94-98, ISSN 0939-6411, https://doi.org/10.1016/j.ejpb.2006.07.011) the development of solid self-emulsifying systems (SSEDDS) through extrusion/spheronization technique is reported. In this work, the possibility of producing self-emulsifying systems was evaluated starting from a liquid emulsion deposited on a solid carrier (microcrystalline cellulose). The system is then subjected to the extrusion/spheronization process. This process allows to obtain pellets (having a size of 1.4 - 2.0 mm) with self-emulsifying capacity, which allow to obtain an increased release of two model molecules characterized by poor solubility in water (methyl-paraben and propyl-paraben).

Based on the state of the art illustrated herein, the inventors understood that the prior art systems left unsolved the problem of making a solid self-emulsifying system capable of creating a liquid emulsion at body temperature. In fact, there are many self-emulsifying liquid systems, transformed into solids via inclusion in solid casings (Cole, E.T. et al. (2008) Challenges and opportunities in the encapsulation of liquid and semi-solid formulations into capsules for oral administration. Adv. Drug. Deliv. Rev. 60, 747-756) or supported on adsorbent solid carriers (WO2003 / 013421), but to date there are no solid systems, at room temperature, which have self-emulsifying capacity when taken into the organism.

The object of the present invention is therefore to overcome the prior art and thus provide a self-emulsifying system that is solid at room temperature, and which is however capable of carrying out its function at a temperature higher than the ambient one, that is, at body temperature.

### SUMMARY OF THE INVENTION

The inventors have therefore surprisingly made a self-emulsifying system that is in a solid state at room temperature but is able to melt and form an emulsion when being at body temperature inside the organism, under the mild stirring provided by peristaltic movements.

The invention therefore concerns a self-emulsifying solid particle at body temperature comprising
- from 50% to 90% by weight, with respect to the weight of the particle, of at least one fatty substance having a melting point in the range from 40°C to 90°C,
- from 5% to 50% by weight, with respect to the weight of the particle, of at least one fatty substance having a melting point lower than 40°C, and
- from 1 to 50%, with respect to the weight of the particle, of at least one emulsifier wherein

said at least one fatty substance having a melting point in the range from 40°C to 90°C is selected from the group consisting of glycerol behenate, stearic acid, magnesium stearate, vegetable stearin, bees wax, carnauba wax and candelilla wax,
said at least one fatty substance having a melting point lower than 40°C is selected from the group consisting of cocoa butter, margarine, butter, olive oil, linseed oil, grape seed oil and medium chain triglycerides (MCT), and
the at least one emulsifier is a polysorbate (Polyoxyethylene Sorbitan Monooleate).

The solid particle of the invention advantageously comprises also from 0.1 to 0.5%, with respect to the weight of the particle, of at least one antioxidant agent.

The solid particle of the invention may advantageously comprise at least one poorly soluble/bioavailable active ingredient.

In the present invention, when the definition of "poorly soluble/bioavailable active ingredient" is used, it is intended to mean an active ingredient falling in the BCS (Biopharmaceutical Classification System) Class 2, 3 or 4, as will be detailed in the following detailed description.

Advantageously, the present invention therefore allows to have solid particles, and thus characterized by all the advantages of handling and workability typical of powders, but capable of forming a stable emulsion when they are at body temperature (36 - 40°C) and are subjected to the mild peristaltic movements typical of the gastrointestinal system. The self-emulsifying solid particles may advantageously comprise at least one active ingredient and therefore may also be defined as microencapsulates which, by their constitution, are solid at room temperature, but due to their formulation peculiarities, the particles object of this invention are characterized by the self-emulsifying capacity, therefore they generate an emulsion, when taken into the body, being at body temperature and under mild stirring caused by peristaltic movements. This ability enables to suspend and/or emulsify the insoluble or poorly soluble active ingredients in the body's aqueous solutions.

Disclosed, but not claimed, is a process for the preparation of at least one self-emulsifying solid particle at body temperature comprising the following steps:
a) preparing an initial formulation by mixing the following ingredients:
   - from 50% to 90% by weight, with respect to the weight of the initial formulation, of at least one fatty substance having a melting point in the range from 40°C to 90°C,
   - from 5% to 50% by weight, with respect to the weight of the initial formulation, of at least one fatty substance having a melting point lower than 40°C, and
   - from 1 to 50%, with respect to the weight of the initial formulation, of at least one emulsifier;
b) melting and homogenizing the initial formulation, thus obtaining a melted mass
d) cooling said melted mass and obtaining at least one self-emulsifying solid particle,
wherein
said at least one fatty substance having a melting point in the range from 40°C to 90°C is selected from the group consisting of glycerol behenate, stearic acid, magnesium stearate, vegetable stearin, bees wax, carnauba wax and candelilla wax,
said at least one fatty substance having a melting point lower than 40°C is selected from the group consisting of cocoa butter, margarine, butter, olive oil, linseed oil, grape seed oil and medium chain triglycerides (MCT), and
the at least one emulsifier is a polysorbate (Polyoxyethylene Sorbitan Monooleate). Advantageously, the process of the invention comprises a step c), between step b) and step d), which provides for the addition of at least one poorly soluble/bioavailable active ingredient.

Preferably, in b), from 0.1 to 0.5% by weight, with respect to the weight of the initial formulation, of at least one antioxidant agent is added.

Disclosed, but not claimed, is the solid particle obtainable from the process disclosed, which is solid at room temperature and self-emulsifying at a body temperature comprised in the range from 36 to 40°C.

Said solid particle of the invention has a median diameter comprised between 125 µm and 250 µm, as measured by particle size analysis through a sieve method using an ENDECOTTS - OCTAGON 200 vibrating screen.

Advantageously, the present invention allows to have solid particles, and thus characterized by all the advantages of handling and workability typical of powders, but capable of forming a stable emulsion when they are at body temperature (36 - 40°C) and are subjected to the mild peristaltic movements typical of the gastrointestinal system. The solid particles may also be defined as microencapsulates which, by their constitution, are solid at room temperature, but due to the formulation peculiarities, the particles object of this invention are characterized by a self-emulsifying capacity, therefore they generate an emulsion, when taken in the organism, being at body temperature and under mild stirring caused by peristaltic movements. This ability allows to suspend and/or emulsify the insoluble or poorly soluble active ingredients in the body's aqueous solutions.

In yet another aspect, the invention relates to a microencapsulate comprising the self-emulsifying solid particle at body temperature, and at least one poorly soluble/bioavailable active ingredient.

As will be apparent from the experimental part that follows, the particle obtainable from the process disclosed, solid at room temperature and self-emulsifying at body temperature in the range from 36 to 40°C, when in the presence of body temperature, is dispersed in intestinal fluids having neutral pH, thus generating micelles having a hydrodynamic diameter in the range from 100 to 900 nm (as measured by dynamic light scattering (DLS)) and which remain stable for up to 180 minutes.

The invention therefore allows to have the administration/delivery of at least one active ingredient as a solid product, characterized by the advantages of this physical state, such as ease of transport, storage and processing (e.g. making tablets, capsules, sachets, stick-packs, etc.), thus allowing to increase the solubility, and therefore the bioavailability, of normally insoluble pharmaceutical or nutraceutical active ingredients characterized by poor body absorption.

In another aspect, therefore, the invention relates to the self-emulsifying solid particle for use in the delivery of active ingredients.

### DESCRIPTION OF THE FIGURES

Figure 1 represents the initial three-phase diagram used for the development of the solid self-emulsifying system. The highlighted part identifies the region of the graph taken into consideration for the study of the system in Example 1.
Figure 2 shows the initial three-phase diagram, shown in Figure 1, with the optimal region obtained thanks to the results of the studies carried out in Example 1 and highlighted with dark gray lines.
Figure 3 shows, on the left, the outcome of the disaggregation test carried out on three control points within the region highlighted in Figure 1; on the right there is a detail of the emulsion that is generated, with its opalescent appearance highlighted.
Figure 4 highlights the aspect related to the stability of the emulsions obtained from three control points evaluated in Example 2. On the left, the appearance of the emulsions after a week of storage is shown. On the right, the same are evaluated after two weeks of storage.
Figure 5 shows the results of the analysis of Example 6 related to the size distribution of sample "21LA03343 - C-1-054-21, self-emulsifying system" by means of DLS after 30 minutes (solid line), 90 minutes (dashed line) and 180 minutes (dotted line) of incubation at 37°C in intestinal fluids.
Figure 6 shows the results of the analysis of Example 11 related to the size distribution of the sample "22LA05496 - C-1-081-22, self-emulsifying system" by means of DLS after 30 minutes (solid line), 90 minutes (dashed line) and 180 minutes (dotted line) of incubation at 37°C in intestinal fluids.
Figure 7 shows the results of the analysis of Example 14 related to the size distribution of the sample "22LA05498 - C-3-081-22, self-emulsifying system" by means of DLS after 30 minutes (solid line), 90 minutes (dashed line) and 180 minutes (dotted line) of incubation at 37°C in intestinal fluids.

### DETAILED DESCRIPTION OF THE INVENTION

The invention therefore concerns a self-emulsifying solid particle at body temperature comprising:
- from 50% to 90% by weight, with respect to the weight of the particle, of at least one fatty substance having a melting point in the range from 40°C to 90°C,
- from 5% to 50% by weight, with respect to the weight of the particle, of at least one fatty substance having a melting point lower than 40°C, and,
- from 1 to 50%, with respect to the weight of the particle, of at least one emulsifier,
wherein
said at least one fatty substance having a melting point in the range from 40°C to 90°C is selected from the group consisting of glycerol behenate, stearic acid, magnesium stearate, vegetable stearin, bees wax, carnauba wax and candelilla wax,
said at least one fatty substance having a melting point lower than 40°C is selected from the group consisting of cocoa butter, margarine, butter, olive oil, linseed oil, grape seed oil and medium chain triglycerides (MCT), and
the at least one emulsifier is a polysorbate (Polyoxyethylene Sorbitan Monooleate).

The solid particle of the invention may advantageously comprise at least one poorly soluble/bioavailable active ingredient.

In the present invention, when the definition of "poorly soluble/bioavailable active ingredient" is used, it is intended to mean an active ingredient falling in the BCS (Biopharmaceutical Classification System) Class 2, 3 or 4.

The active ingredients are divided into BCS (Biopharmaceutical Classification System) classes based on solubility and absorption (MEHTA, Mehul. Biopharmaceutics Classification System (bcs). John Wiley & Sons, 2017):
- Class 1: High solubility and high absorption;
- Class 2: Reduced solubility and high absorption;
- Class 3: High solubility and reduced absorption;
- Class 4: Reduced solubility and reduced absorption.

In the present invention, when the definition of "poorly soluble/bioavailable active ingredient" is used, it is intended to mean an active ingredient falling in the BCS (Biopharmaceutical Classification System) Class 2, 3 or 4.

The self-emulsifying solid particle of the invention comprises from 50% to 90% by weight, with respect to the weight of the particle, of at least one fatty substance having a melting point in the range from 40°C to 90°C, wherein said at least one fatty substance having a melting point in the range from 40°C to 90°C is selected from the group consisting of glycerol behenate, stearic acid, magnesium stearate, vegetable stearin, bees wax, carnauba wax and candelilla wax.

Preferably the fatty substance having a melting point in the range from 40°C to 90°C is in the range from 60% to 80%, more preferably from 60% to 70%, even more preferably from 63% to 66% with respect to the weight of the particle.

Said at least one fatty substance having a melting point in the range from 40°C to 90°C is selected from the group consisting of glycerol behenate, stearic acid, magnesium stearate, vegetable stearin, bees wax, carnauba wax and candelilla wax. In an advantageous and preferred embodiment, it is glycerol behenate.

The solid particle comprises from 5% to 50% by weight, with respect to the weight of the particle, of at least one fatty substance having a melting point lower than 40°C, wherein said at least one fatty substance having a melting point lower than 40°C is selected from the group consisting of cocoa butter, margarine, butter, olive oil, linseed oil, grape seed oil and medium chain triglycerides (MCT). Preferably, the at least one fatty substance having a melting point lower than 40°C is in the range from 10% to 40%, more preferably from 15% to 25%, even more preferably from 18% to 22% by weight, with respect to the weight of the solid particle.

Said at least one fatty substance having a melting point lower than 40°C is preferably selected from the group consisting of cocoa butter, margarine, butter, olive oil, linseed oil, grape seed oil and medium chain triglycerides (MCT), more preferably cocoa butter.

The solid particle also comprises at least one emulsifier in an amount in the range from 1% to 50%. Said amount is preferably in the range from 10 to 30%, more preferably from 15 to 20%, even more preferably from 15 to 19% with respect to the weight of the particle.

According to the invention, the at least one emulsifier is a polysorbate (Polyoxyethylene Sorbitan monooleate). Preferably said at least one emulsifier is selected from the group consisting of Polysorbate 20, Polysorbate 40, Polysorbate 45, Polysorbate 60, Polysorbate 65, Polysorbate 80. More preferably, the emulsifier is Polysorbate 80 commercially known as TWEEN 80.

The solid particle also preferably comprises from 0.1 to 0.5% of at least one antioxidant agent with respect to the weight of the particle. More preferably, said at least one antioxidant agent is in the range from 0.2 to 0.3% by weight, with respect to the weight of the particle. The at least one antioxidant agent is advantageously and preferably Vitamin E. More preferably the at least one antioxidant agent is a mixture of two or more from alpha-tocopherol, beta-tocopherol, gamma-tocopherol, delta-tocopherol.

Advantageously, the present invention thus allows to have solid particles, therefore characterized by all the advantages of handling and workability typical of powders, but capable of forming a stable emulsion when they are at body temperature (36 - 40°C) and are subjected to the mild peristaltic movements typical of the gastrointestinal system. The solid particles may advantageously comprise at least one active ingredient and also be defined as microencapsulates which, by their constitution, are solid at room temperature, but due to the formulation peculiarities, are characterized by a self-emulsifying capacity, therefore they generate an emulsion, when taken in the organism, being at body temperature and under mild stirring caused by peristaltic movements. This ability allows to suspend and/or emulsify the insoluble or poorly soluble active ingredients in the body's aqueous solutions.

The invention therefore concerns a microencapsulate comprising the self-emulsifying solid particle of the invention and one or more poorly soluble/bioavailable active ingredients.

Said at least one active ingredient is preferably a dry, soft and fluid plant extract for nutraceutical use (Annex 1 to the Ministerial Decree of 10 August 2018 on the regulation of the use of plant substances and preparations in food supplements, as updated with the Decree of 9 January 2018 and lastly with Decree of 26 July 2019) and/or pharmaceutical use; a substance permitted for use in food (nutrients and other substances with a nutritional or physiological effect (Revision September 2019) - Ministry of Health)); vitamins and minerals for food use (COMMISSION REGULATION (EC) No. 1170/2009 of 30 November 2009), vitamins for pharmaceutical use; food substances included in the Novel Food regulation (COMMISSION IMPLEMENTING REGULATION (EU) 2017/2470 of 20 December 2017) and of which there is no history of consumption since before 1997 in the country of use; pharmaceutical grade active ingredients characterized by reduced solubility, reduced absorption and/or reduced solubility and absorption.

Disclosed, but not claimed, is a process for the preparation of at least one solid self-emulsifying particle at body temperature comprising the following steps:
a) preparing an initial formulation by mixing the following ingredients:
   - from 50% to 90% by weight, with respect to the weight of the initial formulation, of at least one fatty substance having a melting point in the range from 40°C to 90°C,
   - from 5% to 50% by weight, with respect to the weight of the initial formulation, of at least one fatty substance having a melting point lower than 40°C, and
   - from 1 to 50%, with respect to the weight of the initial formulation, of at least one emulsifier;
b) melting and homogenizing the formulation
d) cooling said melted mass and obtaining at least one self-emulsifying solid particle,
wherein
said at least one fatty substance having a melting point in the range from 40°C to 90°C is selected from the group consisting of glycerol behenate, stearic acid, magnesium stearate, vegetable stearin, bees wax, carnauba wax and candelilla wax,
said at least one fatty substance having a melting point lower than 40°C is selected from the group consisting of cocoa butter, margarine, butter, olive oil, linseed oil, grape seed oil and medium chain triglycerides (MCT), and
the at least one emulsifier is a polysorbate (Polyoxyethylene Sorbitan Monooleate).

Advantageously, the process comprises a step c), between step b) and step d), which provides for the addition of at least one poorly soluble/bioavailable active ingredient.

The process involves a) preparing an initial formulation by mixing the following ingredients:
- from 50% to 90% by weight, with respect to the weight of the initial formulation, of at least one fatty substance having a melting point in the range from 40°C to 90°C,
- from 5% to 50% by weight, with respect to the weight of the initial formulation, of at least one fatty substance having a melting point lower than 40°C, and
- from 1 to 50%, with respect to the weight of the initial formulation, of at least one emulsifier,
wherein
said at least one fatty substance having a melting point in the range from 40°C to 90°C is selected from the group consisting of glycerol behenate, stearic acid, magnesium stearate, vegetable stearin, bees wax, carnauba wax and candelilla wax,
said at least one fatty substance having a melting point lower than 40°C is selected from the group consisting of cocoa butter, margarine, butter, olive oil, linseed oil, grape seed oil and medium chain triglycerides (MCT), and
the at least one emulsifier is a polysorbate (Polyoxyethylene Sorbitan Monooleate).

Preferably the fatty substance having a melting point in the range from 40°C to 90°C is in the range from 60% to 80%, more preferably from 60% to 70%, even more preferably from 63% to 66% with respect to the weight of the initial formulation.

In step a), the initial formulation comprises from 5% to 50% by weight, with respect to the initial formulation, of at least one fat substance having a melting point lower than 40°C selected from the group consisting of cocoa butter, margarine, butter, olive oil, linseed oil, grape seed oil and medium chain triglycerides (MCT). Preferably the at least one fatty substance having a melting point lower than 40°C is in the range from 10% to 40%, more preferably from 15% to 25%, even more preferably from 18% to 22% by weight, with respect to the weight of the initial formulation.

In the initial formulation there is also at least one polysorbate (Polyoxyethylene Sorbitan monooleate) in an amount in the range from 1% to 50%. Said amount is preferably in the range from 10 to 30%, more preferably from 15 to 20%, even more preferably from 15 to 19% with respect to the weight of the initial formulation.

In step b), the final formulation is melted and brought to homogenization. Preferably step b) provides for adding from 0.1 to 0.5% by weight, with respect to the weight of the initial formulation, of at least one antioxidant agent after homogenization and before step c).

More preferably, said at least one antioxidant agent is in the range from 0.2 to 0.3% by weight with respect to the weight of the initial formulation.

Following step b) according to the invention, a melted mass is obtained.

In step d), the melted mass is cooled and at least one self-emulsifying solid particle is obtained.

All the preferred aspects related to the components of the emulsifying solid particle are the same as the ones in the process that allows it to be obtained, and are repeated herein only for completeness purpose of the process of the invention.

Said at least one fatty substance having a melting point in the range from 40°C to 90°C is selected from the group consisting of glycerol behenate, stearic acid, magnesium stearate, vegetable stearin, bees wax, carnauba wax and candelilla wax. In an advantageous and preferred embodiment, it is glycerol behenate.

Said at least one fatty substance having a melting point lower than 40°C is selected from the group consisting of cocoa butter, margarine, butter, olive oil, linseed oil, grape seed oil and medium chain triglycerides (MCT). More preferably it is cocoa butter.

In the initial formulation at least one emulsifier is present with respect to the weight of the initial formulation.

According to the invention, the emulsifier is a polysorbate (Polyoxyethylene Sorbitan Monooleate). Preferably said at least one emulsifier is selected from the group consisting of Polysorbate 20, Polysorbate 40, Polysorbate 45, Polysorbate 60, Polysorbate 65, Polysorbate 80. More preferably, the emulsifier is Polysorbate 80 commercially known as TWEEN 80.

In step b) the final formulation is melted and brought to homogenization. In step b), after homogenization, the process preferably provides for the addition of at least one antioxidant agent in the range from 0.1% to 0.5% by weight, with respect to the weight of the initial formulation. The at least one antioxidant agent is advantageously and preferably Vitamin E. More preferably the at least one antioxidant agent is a mixture of two or more from alpha-tocopherol, beta-tocopherol, gamma-tocopherol, delta-tocopherol.

Advantageously, the process comprises a step c), between step b) and step d), which provides for the addition of at least one active ingredient having a low solubility/bioavailability.

Preferably, therefore, the process allows the delivery and solubility increase of poorly soluble/bioavailable active ingredients.

The process and the solid particle at room temperature and self-emulsifying at body temperature, therefore, allow the delivery and administration of active ingredients, allowing an increase in solubility and absorption.

Step d) relates to cooling the melted mass of step b) or c) to obtain at least one self-emulsifying solid particle.

Surprisingly, the cooling and the peculiarities of the initial formulation allow to obtain a particulate which is solid at room temperature but, thanks to the composition of the melted mass, melts at body temperature (36-40°C) thus allowing the formation of an emulsion by means of the mild peristaltic movements of the body's gastrointestinal system, with a resulting improvement in the solubility of active ingredients included in a category defined as insoluble.

Such step d) may preferably and advantageously take place by cooling said melted mass by means of a spray cooling plant, thus obtaining at least one self-emulsifying solid particle. The spray cooling technique is commonly defined in at least three ways, namely spray cooling, spray chilling or spray congealing. The spray cooling technique inside a refrigerated cell allows to drastically lower the temperature of the particulate matter obtained from the melted mass during the flight time, and thus obtain the self-emulsifying system in the solid state.

In addition to the spray cooling technique, the cooling step d) may also take place through melt granulation and melt extrusion, melt extrusion/spheronization.

Step d) may therefore take place by means of melt granulation. After obtaining the melt, and the optional and preferred addition of at least one active ingredient, the temperature lowering allows to obtain solid granules with self-emulsifying capacity in aqueous medium at body temperature.

Alternatively, step d) may take place by means of melt extrusion/spheronization. In this case the melted mass is forced through a perforated septum under controlled temperature, flow, and pressure. With this technique, an extrudate having well-defined size is obtained, which can be controlled through the working parameters used. The extrudates produced in this way may be transferred out of the plant on a spheronizing plate which allows to obtain a particulate having a spherical shape and with predetermined size.

The process, and thus the solid particle, may include additives.

Among additives to be added before step d), and which are therefore present in the solid particle, dyes, coating agents, flavorings and sweeteners can be mentioned.

Among dyes, titanium dioxide, calcium carbonate and iron oxides can be mentioned. Among glazing agents, hydroxypropylmethylcellulose, sodium alginate, ethylcellulose, polyvinyl alcohol, poly(butylmethacrylate, (2-dimethylaminoethyl) methacrylate, methyl methacrylate) 1: 2: 1, poly(ethyl acrylate, methyl methacrylate) 2 : 1, poly(methacrylic acid, methyl methacrylate) 1: 1, poly(methacrylic acid, ethyl acrylate) 1 : 1, poly(methacrylic acid, methyl methacrylate) 1 : 2, poly(methyl acrylate, methyl methacrylate, methacrylic acid) 7 : 3: 1, poly(ethyl acrylate, methyl methacrylate, trimethylammonioethyl methacrylate chloride) 1: 2 : 0.2, poly(ethyl acrylate, methyl methacrylate, trimethylammonioethyl methacrylate chloride) 1 : 2 : 0.1.

Among aromas, natural aromas and/or synthetic aromas can be mentioned. Among sweeteners, sorbitol, mannitol, xylitol, erythritol, sucralose, stevia, acesulfame K, sodium cyclamate, aspartame, saccharin, dextrose, isomalt, trehalose, inositol, sucrose, fructose, dextrose, glucose, isomaltulose, neohesperidin dihydrochalcone can be mentioned.

Disclosed, but not claimed, is the solid particle obtainable from the process, which is solid at room temperature and self-emulsifying at a body temperature in the range from 36 to 40°C.

The invention therefore consists in making a solid system at room temperature, but which has self-emulsifying capacity at body temperature following melting thereof, and if subjected to mild stirring, due to the peristaltic movements of the gastrointestinal tract.

The solid particles of the invention had a median diameter comprised between 125 µm and 250 µm, as measured by particle size analysis through a sieve method using an ENDECOTTS - OCTAGON 200 vibrating screen.

Without being bound to any theory, the inventors of the present invention believe that the lipophilic matrix, which forms a relevant part of the self-emulsifying solid particle, allows to solubilize insoluble substances in an aqueous environment, thus allowing their emulsification thanks to the presence of emulsifiers in the formulation. Furthermore, the lipophilic matrix of the initial formulation allows the dispersion of substances which, although soluble in an aqueous environment, are not very bioavailable. This system allows, therefore, to obtain suspensions of the substances of interest, favoring their absorption.

The invention therefore allows to obtain the administration/delivery of at least one active ingredient as a solid product, characterized by the advantages of this physical state, such as ease of transport, storage and processing (e.g. making tablets, capsules, sachets, stick-packs, etc.), thus allowing to increase the solubility, and therefore the bioavailability, of normally insoluble active pharmaceutical or nutraceutical ingredients characterized by poor absorption by the body.

In another aspect, therefore, the invention relates to the self-emulsifying solid particle for use in the delivery of active ingredients.

Without being bound to any theory, the inventors believe that the self-emulsifying solid particle loaded with the at least one active ingredient to form the microencapsulate has the following mechanism of action: the active ingredients are solubilized or dispersed in the oily/hydrophobic matrix (i.e. the fatty substance having a melting point in the range from 40°C to 90°C/ the fatty substance having a melting point lower than 40°C, both specifically defined) which forms the system and, when the solid particles are at a temperature equal to or higher than the body temperature, they tend to melt back to the liquid state. The presence of the emulsifier component therefore drives the formation of micelles in which the core is made of the oily/hydrophobic component (i.e. respectively the fatty substance having a melting point in the range from 40°C to 90°C/ the fatty substance having a melting point lower than 40°C, both specifically defined) in which the active ingredients are dissolved or dispersed.

As will be apparent from the experimental part that follows, the solid particles of the invention, when in the presence of body temperature, get dispersed in intestinal fluids with neutral pH generating micelles having a hydrodynamic diameter in the range from 100 to 900 nm (as measured by dynamic light scattering (DLS)) and remain stable for up to 180 minutes.

Disclosed, but not claimed, is the microencapsulate of the invention for use in the treatment of a disease included in the activities of the at least one poorly soluble/bioavailable active ingredient contained in the microencapsulate. Advantageously, the at least one active ingredient is more absorbed, with a better bioavailability of the same.

The invention will now be illustrated by means of examples provided by way of nonlimiting example of the same invention.

### EXPERIMENTAL PART

In the experimental part that follows, the analytical methods mentioned below were used.

Disaggregation: verifies the formation of the emulsion starting from the self-emulsifying system at a temperature equal to body temperature, and subjected to mild stirring to simulate the peristatic movements of the gastrointestinal tract. Dissolution: verifies the release of the active ingredients by the self-emulsifying system.

HPLC/other technique: verifies the loading capacity of the active ingredient by the self-emulsifying solid particle.

DLS: verifies the size of the micelles of the emulsion formed from the self-emulsifying system at a temperature equal to body temperature, and subjected to mild stirring to simulate the peristatic movements of the gastrointestinal tract to evaluate the stability thereof.

The definitions of the acronyms used are given below.
DLS: Dynamic Light Scattering
HPLC: High Performance Liquid Chromatography
BCS: Biopharmaceutical Classification System

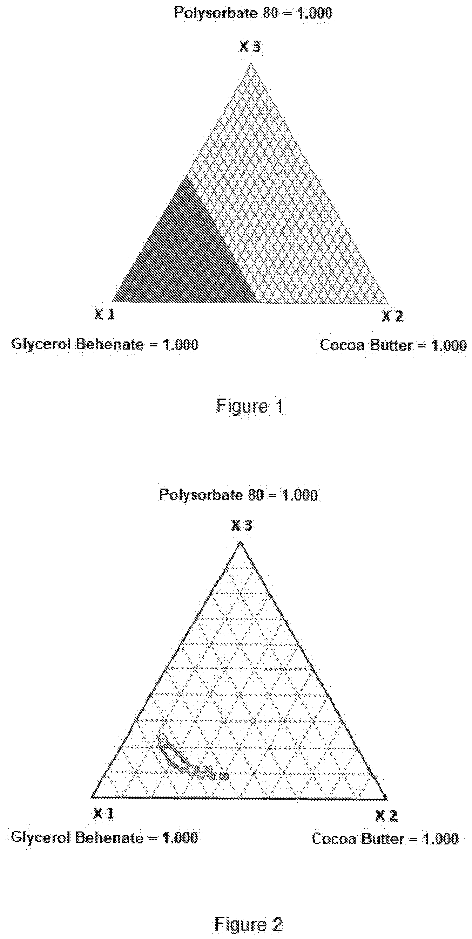

A three-phase diagram was developed (shown in Figure 1) to study the concentrations of the components effective in the preparation of solid particles with self-emulsifying capacity. Examples 1, 2 were carried out within the region highlighted in the three-phase diagram of Figure 1, to evaluate whether the ranges identified for the essential components of the initial formulation allowed the preparation of self-emulsifying solid particles.

### Example 1: Preparation of the self-emulsifying solid particle and evaluation of its components ranges

An amount of Polysorbate 80 equal to 1 g was placed in a water bath at a temperature comprised between 60°C and 90°C. To this, an amount of cocoa butter equal to 24 g was added. Once the latter was completely melted, an amount of glycerol behenate equal to 74.9 g was added to the melted mass. The system was kept under constant stirring throughout the process, until all the components were completely melted and homogenized. An amount of tocopherol mixture (containing alpha-, beta-, gamma- and delta-tocopherol) equal to 0.1 g was added to the melted mass. The melted mass thus obtained was then re-solidified on a surface at room temperature (about 25°C and 60% relative humidity (RH)). Subsequently, the solid obtained was reduced into fragments of homogeneous size (about 1 - 5 mm). A known amount of these fragments (between 1 g and 3 g) was subjected to disaggregation test to verify the actual formation of the emulsion. The SOTAX DT3 disaggregation tester was selected as the initial analysis tool, because it allowed the system to be placed in water under conditions similar to those of the body, i.e. at a pH comprised between 6.5 and 8.5 and at a well-defined temperature (comprised between 36.5°C and 37.5°C), and to replicate the mild stirring provided by the gastrointestinal peristaltic movements. The formation of an opalescent and milky dispersion was taken into consideration as a condition to define whether the emulsion had formed or not.

The fragments obtained in Example 1 had an optimal mechanical strength and, when placed in the disaggregation tester, showed the formation of an emulsion. The opalescent dispersion, exposed to a light source, showed homogeneous diffusion of light, characteristic of systems with presence of emulsion.

### Example 2: Preparation of the self-emulsifying solid particle and evaluation of its components ranges

An amount of Polysorbate 80 equal to 19.75 g was placed in a water bath at a temperature comprised between 60°C and 90°C. To this, an amount of cocoa butter equal to 20 g was added. Once the latter was completely melted, an amount of glycerol behenate equal to 60 g was added to the melted mass. The system was kept under constant stirring throughout the process, until all the components were completely melted and homogenized. An amount of tocopherol mixture (containing alpha-, beta-, gamma- and delta-tocopherol) equal to 0.25 g was added to the melted mass. The melted mass thus obtained was then re-solidified on a surface at room temperature (about 25°C and 60% RH). Subsequently, the solid obtained was reduced into fragments of homogeneous size (about 1 - 5 mm). A known amount of these fragments (between 1 g and 3 g) was subjected to disaggregation test to verify the actual formation of an emulsion. As in Example 1, the SOTAX DT3 disaggregation tester was selected as the initial analysis tool, because it allowed the system to be placed in water with a pH comprised between 6.5 and 8.5 at a well-defined temperature (comprised between 36.5°C and 37.5°C) and to replicate the mild stirring provided by the gastrointestinal peristaltic movements. The formation of an opalescent and milky dispersion was taken into consideration as a condition to define whether the emulsion had formed or not.

The fragments obtained in Example 2 have good mechanical strength and, when placed in the SOTAX DT3 disaggregation tester, show the formation of an emulsion. The opalescent dispersion, exposed to a light source, showed homogeneous diffusion of light, characteristic of systems with presence of emulsion.

With Example 1, both the confirmation of the nature of the essential components and also their formulation ranges were obtained.

Specifically, the initial formulation and therefore the solid particle of the invention comprised:
- from 50% to 90% by weight of at least one fatty substance having a melting point greater than or equal to 40°C, in the specific case glycerol behenate
- from 5% to 50% by weight of at least one fatty substance with a melting point below 40°C, i.e. cocoa butter and
- from 1 to 50% of at least one emulsifier, Polysorbate 80
- from 0.1% to 0.5% of an antioxidant, a mixture of tocopherols (containing alpha-, beta-, gamma- and delta-tocopherol))

Example 1 and Example 2 performed within these ranges led to the formation of systems with self-emulsifying capacity, which remain solid at room temperature. Through the evaluation of points within the above ranges for performance in terms of emulsifying capacity, mechanical strength and handling of the particles obtained, a narrower range was then identified within which the particles obtained had optimal strength characteristics in terms of mechanical strength and self-emulsifying capacity at body temperature.

It corresponds to
- from 63% to 66% by weight of at least one fatty substance having a melting point greater than or equal to 40°C, in the specific case glycerol behenate
- 18% to 22% by weight of at least one fatty substance with a melting point below 40°C, *i.e*. cocoa butter and
- from 15% to 19% of at least one emulsifier, Polysorbate 80
- from 0.2% to 0.3% of an antioxidant, a mixture of tocopherols (containing alpha-, beta-, gamma- and delta-tocopherol).

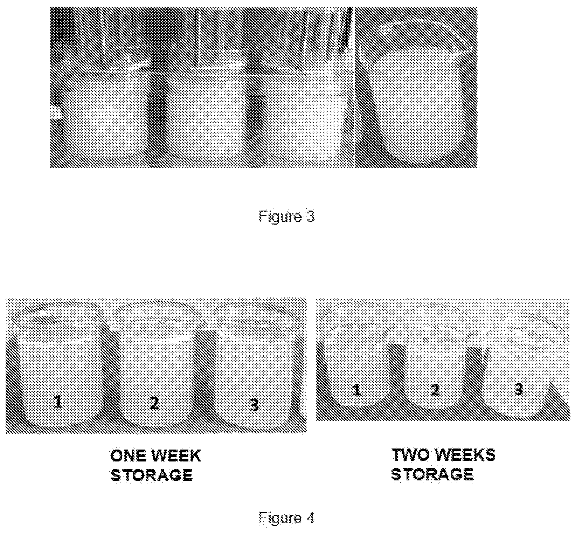

A representation of the results obtained with Experiments 1 and 2 is shown in Figure 3. Figure 3 shows, on the left, the result of the disaggregation test carried out on three control points within the region highlighted in Figure 1; on the right there is a detail of the emulsion that is generated, highlighting the opalescent appearance thereof.

Examples 3, 4 and 5 below are representative of the optimal experimental space identified in Figure 2.

### Example 3

An amount of Polysorbate 80 equal to 18.7 g was placed in a water bath at a temperature comprised between 60°C and 90°C. To this, an amount of cocoa butter equal to 18.3 g was added. Once the latter was completely melted, an amount of glycerol behenate equal to 62.85 g was added to the melted mass. The system was kept under constant stirring throughout the process, until all the components were completely melted and homogenized. An amount of tocopherol mixture (containing alpha-, beta-, gamma- and delta-tocopherol) equal to 0.25 g was added to the melted mass. The melted mass thus obtained was then sprayed by using an airbrush inside a thermostatic chamber at 4°C. The airbrush was selected for spraying the self-emulsifying system, as it simulated the working conditions of the spray cooler used at the production level. This procedure led to the formation of a particulate having particles with a median diameter comprised between 125 µm and 250 µm, as measured by particle size analysis through a sieve method using an ENDECOTTS - OCTAGON 200 vibrating screen. The sieves used were the following: 45 µm, 63 µm, 125 µm, 250 µm, 500 µm, 710 µm, 1000 µm, 2000 µm.

### Example 4

An amount of Polysorbate 80 equal to 15.1 g was placed in a water bath at a temperature comprised between 60°C and 90°C. To this, an amount of cocoa butter equal to 18.3 g was added. Once the latter was completely melted, an amount of glycerol behenate equal to 66.45 g was added to the melted mass. The system was kept under constant stirring throughout the process, until all the components were completely melted and homogenized. An amount of tocopherol mixture (containing alpha-, beta-, gamma- and delta-tocopherol) equal to 0.25 g was added to the melted mass. The melted mass thus obtained was then sprayed by using an airbrush inside a thermostatic chamber at 4°C. This process led to the formation of a particulate having particles with a median diameter comprised between 125 µm and 250 µm, as measured by particle size analysis through a sieve method using an ENDECOTTS - OCTAGON 200 vibrating screen. The sieves used were the following: 45 µm, 63 µm, 125 µm, 250 µm, 500 µm, 710 µm, 1000 µm, 2000 µm.

### Example 5

An amount of Polysorbate 80 equal to 15.1 g was placed in a water bath at a temperature comprised between 60°C and 90°C. To this, an amount of cocoa butter equal to 21.9 g was added. Once the latter was completely melted, an amount of glycerol behenate equal to 62.85 g was added to the melted mass. The system was kept under constant stirring throughout the process, until all the components were completely melted and homogenized. An amount of tocopherol mixture (containing alpha-, beta-, gamma- and delta-tocopherol) equal to 0.25 g was added to the melted mass. The melted mass thus obtained was then sprayed by using an airbrush inside a thermostatic chamber at 4°C. This process led to the formation of particulate matter having particles with median diameter comprised between 125 µm and 250 µm, as measured by particle size analysis through a sieve method using an ENDECOTTS - OCTAGON 200 vibrating screen. The sieves used were the following: 45 µm, 63 µm, 125 µm, 250 µm, 500 µm, 710 µm, 1000 µm, 2000 µm. The particulate obtained in experiments 3, 4 and 5 was subsequently subjected to disaggregation test to evaluate its emulsifying capacity. The analysis parameters were kept the same for all three experiments described above: an amount comprised between 1 g and 3 g of particulate was placed in a SOTAX DT3 disaggregation tester in a solution having a pH comprised between 6.5 and 8.5 and at a temperature comprised between 36.5°C and 37.5°C. The formation of an opalescent and milky dispersion and the evaluation of the homogeneous dispersion of the light source in the system containing the emulsion were taken into consideration as conditions to define whether the emulsion had formed or not. All 3 particulates obtained in experiments 3, 4 and 5 proved effective in generating an emulsion. Furthermore, the emulsions thus obtained were monitored over time to evaluate their stability. The emulsions were kept at a temperature comprised between 38°C and 42°C in an oven, this to avoid the skimming of the emulsion due to system cooling.

Figure 4 shows the three emulsions obtained from experiments 3, 4 and 5. The results obtained show that the solid system obtained has self-emulsifying capacity, if placed in an aqueous environment at neutral pH at a temperature compatible with that of the body. Furthermore, again in Figure 4, it can be seen how the emulsions obtained are stable for a much longer time than the residence time necessary to allow the absorption of the active ingredients in the human body.

### Example 6

Dispersibility analysis of the particle of the invention

The particles of the invention, once dispersed in the body fluid, appeared as a micellar fluid. The micelles were evaluated to determine their size distribution by dynamic light scattering (DLS) when introduced into body fluids.

A sample was prepared according to Example 3.

The sample was indexed as in Table 1 below:

**Table 1 Sample description and acceptance codes assianed to the samples.**

| **Acceptance code** | **Description** |
|---|---|
| 21LA03343 | C-1-054-21, self-emulsifying system |

The sample was dispersed in simulated intestinal fluids (SIF) according to the model of Minekus et al. (2014), whose composition is reported in Table 2.

**Table 2 Composition of simulated intestinal fluids**

| | **Concentration (mmol/L)** |
|---|---|
| **KCl** | 5.44 |
| **KH₂PO₄** | 0.64 |
| **NaHCO₃** | 68 |
| **NaCl** | 30.7 |
| **MgCl₂˙6H₂O** | 0.26 |
| **CaCl₂˙2H₂O** | 0.48 |
| **HCl (for pH correction)** | 6.72 |

The powder sample (21LA03343) was dispersed in SIF at a final concentration of 1 mg/mL.

The particles of the invention dispersed forming micelles, and the dispersions of the micelles were incubated under gentle stirring at 37°C and collected and analyzed after 30 min, 90 min and 180 min. The micelles size distribution obtained by DLS was evaluated on at least 5 replicated measurements, as a function of the signal intensity alone.

### Results

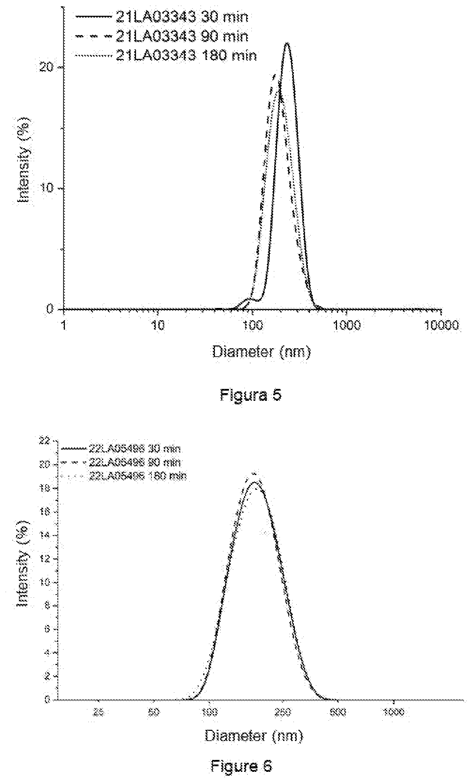

The sample "21LA03343 - C-1-054-21, self-emulsifying system", under the conditions of analysis had a single peak, as shown in Figure 5. The hydrodynamic diameter of the micelles measured in the sample "21LA03343 - C-1-054-21, self-emulsifying system" after 30-90-180 minutes of incubation in SIF is reported in Table 3 below. The values are accompanied by the standard deviation (SD, t= 30 min n=9, t= 90 min n=15, t= 180 min n=12) and the relative standard deviation expressed as a percentage (RSD). Table 3 also shows the polydispersity index of the suspension (Pdl) with standard deviation (SD, t= 30 min n=9, t= 90 min n=15, t= 180 min n=12) and relative standard deviation expressed as a percentage (RSD).

**Table 3**

| **21LA03343** | **Peak 1** | | | **Polydispersity Index** | | |
|---|---|---|---|---|---|---|
| **Time** | **Diameter (nm)** | **SD (nm)** | **RSD (%)** | **PdI** | **SD** | **RSD (%)** |
| **30 min** | 278 | 28 | 10 | 0.39 | 0.05 | 12 |
| **90 min** | 196 | 23 | 12 | 0.23 | 0.03 | 12 |
| **180 min** | 204 | 16 | 8 | 0.23 | 0.03 | 14 |

With reference to Figure 5, after 90 minutes, the micelle peak was seen to shift towards a smaller size. The micelles reached their stability by reducing in size and stabilizing around 200 nm. The Pdl decreased with the passage of time indicating an increase in the stability of the emulsion. This index (Pdl) is comprised between O and 1, the closer it is to 0 the more the emulsion is monodisperse; for indices equal to 1, instead, the emulsion is considered totally polydisperse and therefore more unstable. The sample of the invention was therefore found to form a very stable monodisperse emulsion.

From the data obtained, it was therefore clear that the "C-1-054-21, self-emulsifying system" was dispersed in intestinal fluids with neutral pH, generating micelles of approximately 200 nm in size which were stable up to at least 180 minutes.

### Example 7: Preparation of the self-emulsifying solid particle and evaluation of its components ranges

An amount of Polysorbate 80 equal to 25 g was placed in a water bath at a temperature comprised between 60°C and 90°C. To this, an amount of Cocoa Butter equal to 9 g was added. Once the latter was completely melted, an amount of glycerol behenate equal to 50 g was added to the melted mass. The system was kept under constant stirring throughout the process, until all the components were completely melted and homogenized. The melted mass thus obtained was then re-solidified on a surface at room temperature (about 25°C and 60% relative humidity (RH)). Subsequently, the solid obtained was reduced into fragments of homogeneous size (about 1 - 5 mm). A known amount of these fragments (between 1 g and 3 g) was subjected to disaggregation test to verify the actual formation of the emulsion. The SOTAX DT3 disaggregation tester was selected as the initial analysis tool, because it allowed the system to be placed in water under conditions similar to those of the body, i.e. at a pH comprised between 6.5 and 8.5 and at a well-defined temperature (comprised between 36.5°C and 37.5°C), and to replicate the mild stirring provided by the gastrointestinal peristaltic movements. The formation of an opalescent and milky dispersion was taken into consideration as a condition to define whether the emulsion had formed or not.

The fragments obtained from Example 7, placed in the disaggregation tester, showed the formation of an emulsion.

The opalescent dispersion, exposed to a light source, showed homogeneous diffusion of light, characteristic of systems with presence of emulsion.

### Example 8: Preparation of the self-emulsifying solid particle and evaluation of its components ranges

An amount of Polysorbate 80 equal to 33 g was placed in a water bath at a temperature comprised between 60°C and 90°C. To this, an amount of cocoa butter equal to 8 g was added. Once the latter was completely melted, an amount of glycerol behenate equal to 58 g was added to the melted mass. The system was kept under constant stirring throughout the process, until all the components were completely melted and homogenized. The melted mass thus obtained was then re-solidified on a surface at room temperature (about 25°C and 60% relative humidity (RH)). Subsequently, the solid obtained was reduced into fragments of homogeneous size (about 1 - 5 mm). A known amount of these fragments (between 1 g and 3 g) was subjected to disaggregation test to verify the actual formation of the emulsion. The SOTAX DT3 disaggregation tester was selected as the initial analysis tool, because it allowed the system to be placed in water under conditions similar to those of the body, i.e. at a pH comprised between 6.5 and 8.5 and at a well-defined temperature (comprised between 36.5°C and 37.5°C), and to replicate the mild stirring provided by the gastrointestinal peristaltic movements. The formation of an opalescent and milky dispersion was taken into consideration as a condition to define whether the emulsion had formed or not.

The fragments obtained from Example 8, placed in a disaggregation tester, showed the formation of an emulsion.

The opalescent dispersion, exposed to a light source, showed homogeneous diffusion of light, characteristic of systems with presence of emulsion.

### Example 9: Preparation of the self-emulsifying solid particle and evaluation of its components ranges

An amount of Polysorbate 80 equal to 8 g was placed in a water bath at a temperature comprised between 60°C and 90°C. To this, an amount of cocoa butter equal to 33 g was added. Once the latter was completely melted, an amount of glycerol behenate equal to 58 g was added to the melted mass. The system was kept under constant stirring throughout the process, until all the components were completely melted and homogenized. The melted mass thus obtained was then re-solidified on a surface at room temperature (about 25°C and 60% relative humidity (RH)). Subsequently, the solid obtained was reduced into fragments of homogeneous size (about 1 - 5 mm). A known amount of these fragments (between 1 g and 3 g) was subjected to disaggregation test to verify the actual formation of the emulsion. The SOTAX DT3 disaggregation tester was selected as the initial analysis tool, because it allowed the system to be placed in water under conditions similar to those of the body, i.e. at a pH comprised between 6.5 and 8.5 and at a well-defined temperature (comprised between 36.5°C and 37.5°C), and to replicate the mild stirring provided by the gastrointestinal peristaltic movements. The formation of an opalescent and milky dispersion was taken into consideration as a condition to define whether the emulsion had formed or not.

The fragments obtained from Example 9, placed in a disaggregation tester, showed the formation of an emulsion.

The opalescent dispersion, exposed to a light source, showed homogeneous diffusion of light, characteristic of systems with presence of emulsion.

### Example 10: Preparation of the self-emulsifying solid particle and evaluation of its components ranges

An amount of Polysorbate 80 equal to 8 g was placed in a water bath at a temperature comprised between 60°C and 90°C. To this, an amount of cocoa butter equal to 12 g was added. Once the latter was completely melted, an amount of glycerol behenate equal to 80 g was added to the melted mass. The system was kept under constant stirring throughout the process, until all the components were completely melted and homogenized. The melted mass thus obtained was then re-solidified on a surface at room temperature (about 25°C and 60% relative humidity (RH)). Subsequently, the solid obtained was reduced into fragments of homogeneous size (about 1 - 5 mm). A known amount of these fragments (between 1 g and 3 g) was subjected to disaggregation test to verify the actual formation of the emulsion. The SOTAX DT3 disaggregation tester was selected as the initial analysis tool, because it allowed the system to be placed in water under conditions similar to those of the body, *i.e*. at a pH comprised between 6.5 and 8.5 and at a well-defined temperature (comprised between 36.5°C and 37.5°C), and to replicate the mild stirring provided by the gastrointestinal peristaltic movements. The formation of an opalescent and milky dispersion was taken into consideration as a condition to define whether the emulsion had formed or not.

The fragments obtained in Example 10, placed in a disaggregation tester, showed the formation of an emulsion.

The opalescent dispersion, exposed to a light source, showed homogeneous diffusion of light, characteristic of systems with presence of emulsion.

### Example 11: Dispersibility analysis of the particle of the invention

The particles of the invention, once dispersed in the body fluid, appeared as a micellar fluid. The micelles were evaluated to determine their size distribution by dynamic light scattering (DLS) when introduced into body fluids.

A sample was prepared according to Example 10.

The sample was indexed as in Table 4 below:

**Table 4 Sample description and acceptance codes assigned to the samples.**

| **Acceptance code** | **Description** |
|---|---|
| 22LA05496 | C-1-081-22, self-emulsifying system |

The sample was dispersed in simulated intestinal fluids (SIF) according to the model of Minekus et al. (2014) whose composition is reported in Table 5.

**Table 5 Composition of simulated intestinal fluids**

| | **Concentration (mmol/L)** |
|---|---|
| **KCl** | 5.40 |
| **KH₂PO₄** | 0.64 |
| **NaHCO₃** | 68 |
| **NaCl** | 30 |
| **MgCl₂˙6H₂O** | 0.25 |
| **CaCl₂˙2H₂O** | 0.47 |
| **HCl (for pH correction)** | 2.9 |

The powder sample (22LA05496) was dispersed in SIF at a final concentration of 1 mg/mL.

The particles of the invention dispersed forming micelles, and the dispersions of the micelles were incubated under gentle stirring at 37°C and collected and analyzed after 30 min, 90 min and 180 min. The size distribution of the micelles obtained by DLS was evaluated on at least 5 replicated measurements, as a function of the signal intensity alone.

### Results

The sample "22LA05496- C-1-081-22, self-emulsifying system", under the conditions of analysis had a single peak, as shown in Figure 6. The hydrodynamic diameter of the micelles measured in the sample "22LA05496 - C-1-081-22, self-emulsifying system" after 30-90-180 minutes of incubation in SIF is reported in Table 6 below. The values are accompanied by the standard deviation (SD, t= 30 min n=5, t= 90 min n=5, t= 180 min n=5) and the relative standard deviation expressed as a percentage (RSD). Table 6 also shows the polydispersity index of the suspension (Pdl) with standard deviation (SD, t= 30 min n=5, t= 90 min n=5, t= 180 min n=5) and relative standard deviation expressed as a percentage (RSD).

**Table 6**

| **22LA05496** | **Peak** | | | **Polydispersity Index** | | |
|---|---|---|---|---|---|---|
| **Time** | **Diameter (nm)** | **SD (nm)** | **RSD (%)** | **PdI** | **SD** | **RSD (%)** |
| **30 min** | 187 | 3 | 1.6 | 0.221 | 0.003 | 1.4 |
| **90 min** | 183 | 12 | 6.6 | 0.30 | 0.05 | 17 |
| **180 min** | 186 | 8 | 4.3 | 0.29 | 0.03 | 10 |

With reference to Figure 6, the micelle peak was seen to remain at constant size up to 180 min. The micelles showed their stability already at 30 min by stabilizing around a size of about 200 nm. The Pdl remained unchanged with the passage of time, indicating stability of the emulsion. Such index (Pdl) is comprised between 0 and 1, the closer it is to 0 the more the emulsion is monodisperse; for indices equal to 1, instead, the emulsion is considered totally polydisperse and therefore more unstable. The sample of the invention was therefore found to form a very stable monodisperse emulsion.

From the data obtained, it was therefore clear that the "C-1-081-22, self-emulsifying system" was dispersed in intestinal fluids with neutral pH, generating micelles of approximately 200 nm in size, which were stable up to at least 180 minutes.

### Example 12: Preparation of the self-emulsifying solid particle and evaluation of its components

An amount of Polysorbate 80 equal to 20 g was placed in a water bath at a temperature comprised between 60°C and 90°C. To this, an amount of olive oil equal to 10 g was added. Once the latter was completely melted, an amount of stearic acid equal to 70 g was added to the melted mass. The system was kept under constant stirring throughout the process, until all the components were completely melted and homogenized. The melted mass thus obtained was then re-solidified on a surface at room temperature (about 25°C and 60% relative humidity (RH)). Subsequently, the solid obtained was reduced into fragments of homogeneous size (about 1 - 5 mm). A known amount of these fragments (between 1 g and 3 g) was subjected to disaggregation test to verify the actual formation of the emulsion. The SOTAX DT3 disaggregation tester was selected as the initial analysis tool, because it allowed the system to be placed in water under conditions similar to those of the body, *i.e*. at a pH comprised between 6.5 and 8.5 and at a well-defined temperature (comprised between 36.5°C and 37.5°C), and to replicate the mild stirring provided by the gastrointestinal peristaltic movements. The formation of an opalescent and milky dispersion was taken into consideration as a condition to define whether the emulsion had formed or not.

The fragments obtained in Example 12, placed in a disaggregation tester, showed the formation of an emulsion.

The opalescent dispersion, exposed to a light source, showed homogeneous diffusion of light, characteristic of systems with presence of emulsion.

### Example 13: Preparation of the self-emulsifying solid particle and evaluation of its components

An amount of Polysorbate 80 equal to 10 g was placed in a water bath at a temperature comprised between 60°C and 90°C. To this, an amount of medium chain triglycerides equal to 15 g was added. Once the latter was completely melted, an amount of Candelilla wax equal to 75 g was added to the melted mass. The system was kept under constant stirring throughout the process, until all the components were completely melted and homogenized. The melted mass thus obtained was then re-solidified on a surface at room temperature (about 25°C and 60% relative humidity (RH)). Subsequently, the solid obtained was reduced into fragments of homogeneous size (about 1 - 5 mm). A known amount of these fragments (between 1 g and 3 g) was subjected to disaggregation test to verify the actual formation of the emulsion. The SOTAX DT3 disaggregation tester was selected as the initial analysis tool, because it allowed the system to be placed in water under conditions similar to those of the body, *i.e*. at a pH comprised between 6.5 and 8.5 and at a well-defined temperature (comprised between 36.5°C and 37.5°C), and to replicate the mild stirring provided by the gastrointestinal peristaltic movements. The formation of an opalescent and milky dispersion was taken into consideration as a condition to define whether the emulsion had formed or not. The fragments obtained from Example 13, placed in the disaggregation tester, showed the formation of an emulsion.

The opalescent dispersion, exposed to a light source, showed homogeneous diffusion of light, characteristic of systems with presence of emulsion.

### Example 14: Dispersibility analysis of the particle of the invention

The particles of the invention, once dispersed in the body fluid, appeared as a micellar fluid. The micelles were evaluated to determine their size distribution by dynamic light scattering (DLS) when introduced into body fluids.

A sample was prepared according to Example 13

The sample was indexed as in Table 7 below:

**Table 7 Sample description and acceptance codes assigned to the samples.**

| **Acceptance Code** | **Description** |
|---|---|
| 22LA05498 | C-3-081-22, self-emulsifying system |

The sample was dispersed in simulated intestinal fluids (SIF) according to the model of Minekus et al. (2014), whose composition is reported in Table 5.

The powder sample (22LA05498) was dispersed in SIF at a final concentration of 1 mg/mL.

The particles of the invention dispersed forming micelles, and the dispersions of the micelles were incubated under gentle stirring at 37°C and collected and analyzed after 30 min, 90 min and 180 min. The size distribution of the micelles obtained by DLS was evaluated on at least 5 replicated measurements, as a function of the signal intensity alone.

### Results

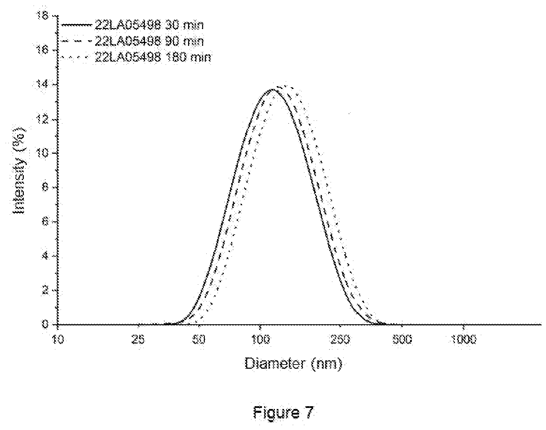

The sample "22LA05498 - C-3-081-22, self-emulsifying system", under the test conditions had a single peak, as shown in Figure 7. The hydrodynamic diameter of the micelles measured in the sample "22LA05498 - C-3-081-22, self-emulsifying system" after 30-90-180 minutes of incubation in SIF is reported in Table 8 below. The values are accompanied by the standard deviation (SD, t= 30 min n=5, t= 90 min n=5, t= 180 min n=5) and the relative standard deviation expressed as a percentage (RSD). Table 8 also shows the polydispersity index of the suspension (Pdl) with standard deviation (SD, t= 30 min n=5, t= 90 min n=5, t= 180 min n=5) and relative standard deviation expressed as a percentage (RSD).

**Table 8**

| **22LA05498** | **Peak** | | | **Polydispersity Index** | | |
|---|---|---|---|---|---|---|
| **Time** | **Diameter (nm)** | **SD (nm)** | **RSD (%)** | **PdI** | **SD** | **RSD (%)** |
| **30 min** | 125 | 2 | 1.6 | 0.14 | 0.01 | 7 |
| **90 min** | 133 | 8 | 6 | 0.16 | 0.03 | 19 |
| **180 min** | 146 | 1 | 0.7 | 0.149 | 0.006 | 4 |

With reference to Figure 7, it was seen that, after 90 minutes, the micelle peak shifted to a larger size. The micelles reached their stability by increasing in size and stabilizing around 150 nm. The Pdl remained unchanged with the passage of time, indicating a stability of the emulsion. This index (Pdl) is between 0 and 1, the closer it is to 0 the more the emulsion is monodispersed; for indices equal to 1, instead, the emulsion is considered totally polydisperse and therefore more unstable. The sample of the invention was therefore found to form a very stable monodisperse emulsion.

From the data obtained it was therefore clear that the "C-3-081-22, self-emulsifying system" was dispersed in intestinal fluids with neutral pH generating micelles of approximately 150 nm size which were stable up to at least 180 minutes.

### Example 15: Preparation of the self-emulsifying solid particle and evaluation of its components

An amount of Polysorbate 80 equal to 15 g was placed in a water bath at a temperature comprised between 60°C and 90°C. To this, an amount of cocoa butter equal to 10 g was added. Once the latter was completely melted, an amount of glycerol stearate equal to 75 g was added to the melted mass. The system was kept under constant stirring throughout the process, until all the components were completely melted and homogenized. The melted mass thus obtained was then re-solidified on a surface at room temperature (about 25°C and 60% relative humidity (RH)). Subsequently, the solid obtained was reduced into fragments of homogeneous size (about 1 - 5 mm). A known amount of these fragments (between 1 g and 3 g) was subjected to disaggregation test to verify the actual formation of the emulsion. The SOTAX DT3 disaggregation tester was selected as the initial analysis tool, because it allowed the system to be placed in water under conditions similar to those of the body, *i.e*. at a pH comprised between 6.5 and 8.5 and at a well-defined temperature (comprised between 36.5°C and 37.5°C), and to replicate the mild stirring provided by the gastrointestinal peristaltic movements. The formation of an opalescent and milky dispersion was taken into consideration as a condition to define whether the emulsion had formed or not.

The fragments obtained in Example 15, placed in a disaggregation tester, showed the formation of an emulsion.

The opalescent dispersion, exposed to a light source, showed homogeneous diffusion of light, characteristic of systems with presence of emulsion.

## Claims

1. A self-emulsifying solid particle at body temperature comprising:
- from 50% to 90% by weight, with respect to the weight of the particle, of at least one fatty substance having a melting point in the range from 40°C to 90°C,
- from 5% to 50% by weight, with respect to the weight of the particle, of at least one fatty substance having a melting point lower than 40°C, and
- from 1 to 50%, with respect to the weight of the particle, of at least one emulsifier,
wherein
said at least one fatty substance having a melting point in the range from 40°C to 90°C is selected from the group consisting of glycerol behenate, stearic acid, magnesium stearate, vegetable stearin, bees wax, carnauba wax and candelilla wax,
said at least one fatty substance having a melting point lower than 40°C is selected from the group consisting of cocoa butter, margarine, butter, olive oil, linseed oil, grape seed oil and medium chain triglycerides (MCT), and the at least one emulsifier is a polysorbate (Polyoxyethylene Sorbitan Monooleate).

2. The self-emulsifying solid particle of claim 1, wherein said particle comprises at least one active ingredient falling within the BCS (Biopharmaceutical Classification System) Class 2, 3 or 4.

3. The self-emulsifying solid particle of claim 1 or 2, wherein said solid particle comprises from 0.1 to 0.5%, with respect to the weight of the particle, of at least one antioxidant agent, preferably said at least one antioxidant agent is in the range from 0.2 to 0.3% by weight, with respect to the weight of the particle.

4. The self-emulsifying solid particle according to any one of claims 1 to 3, wherein the fatty substance having a melting point in the range from 40°C to 90°C is in the range from 60% to 80%, preferably from 60% to 70%, more preferably from 63% to 66% with respect to the weight of the particle.

5. The self-emulsifying solid particle according to any one of claims 1 to 4, wherein said at least one fatty substance having a melting point in the range from 40°C to 90°C is glycerol behenate.

6. The self-emulsifying solid particle according to any one of claims 1 to 5, wherein the at least one fatty substance having a melting point lower than 40°C is in the range from 10% to 40%, preferably from 15% to 25%, more preferably from 18% to 22% by weight, with respect to the weight of the solid particle.

7. The self-emulsifying solid particle according to any one of claims 1 to 6, wherein said at least one fatty substance having a melting point lower than 40°C is cocoa butter.

8. The self-emulsifying solid particle according to any one of claims 1 to 7, wherein the at least one emulsifier is in the range from 10 to 30%, preferably from 15 to 20%, more preferably from 15 to 19%, with respect to the weight of the particle.

9. The self-emulsifying solid particle according to claims 1 to 8, wherein the at least one emulsifier is selected from the group consisting of Polysorbate 20, Polysorbate 40, Polysorbate 45, Polysorbate 60, Polysorbate 65, Polysorbate 80, preferably the emulsifier is Polysorbate 80.

10. The self-emulsifying solid particle according to any one of claims 1 to 9, wherein the at least one antioxidant agent is Vitamin E, preferably the at least one antioxidant agent is a mixture of two or more from alpha-tocopherol, beta-tocopherol, gamma-tocopherol, delta-tocopherol.

11. The self-emulsifying solid particle according to any one of claims 1 to 10, having a median diameter comprised between 125 µm and 250 µm, as measured by particle size analysis through a sieve method using an ENDECOTTS - OCTAGON 200 vibrating screen.

12. The self-emulsifying solid particle according to any one of claims 1 to 11, wherein said particle is solid at room temperature and self-emulsifying at a body temperature comprised in the range from 36 to 40°C.

13. The self-emulsifying solid particle according to any one of claims 1 to 12, wherein said solid particle, in the presence of a body temperature comprised in the range from 36 to 40°C, is capable of self-dispersing in intestinal fluids with neutral pH generating micelles having a hydrodynamic diameter in the range from 100 to 900 nm as measured by dynamic light scattering (DLS) and being stable up to 180 minutes.

14. A microencapsulate comprising the self-emulsifying solid particle of claims 1, 3-13 and one or more active ingredients falling into BCS (Biopharmaceutical Classification System) Class 2, 3 or 4.

15. A self-emulsifying solid particle according to any one of claims 1,3-13 for use in the delivery of one or more active ingredients falling in the BCS (Biopharmaceutical Classification System) Class 2, 3 or 4.

## Patentansprüche

1. Bei Körpertemperatur selbstemulgierendes festes Partikel, umfassend:
- 50 bis 90 Gew.-%, bezogen auf das Gewicht des Partikels, mindestens einer Fettsubstanz mit einem Schmelzpunkt im Bereich von 40 °C bis 90 °C,
- 5 bis 50 Gew.-%, bezogen auf das Gewicht des Partikels, mindestens einer Fettsubstanz mit einem Schmelzpunkt von weniger als 40 °C, und
- 1 bis 50 %, bezogen auf das Gewicht des Partikels, mindestens eines Emulgators, wobei
die mindestens eine Fettsubstanz mit einem Schmelzpunkt im Bereich von 40 °C bis 90 °C ausgewählt ist aus der Gruppe, bestehend aus Glycerinbehenat, Stearinsäure, Magnesiumstearat, pflanzlichem Stearin, Bienenwachs, Carnaubawachs und Candelillawachs,
die mindestens eine Fettsubstanz mit einem Schmelzpunkt von weniger als 40 °C ausgewählt ist aus der Gruppe, bestehend aus Kakaobutter, Margarine, Butter, Olivenöl, Leinöl, Traubenkernöl und mittelkettigen Tryglyceriden (MCT), und der mindestens eine Emulgator ein Polysorbat (Polyoxyethylensorbitanmonooleat) ist.

2. Selbstemulgierendes festes Partikel nach Anspruch 1, wobei das Partikel mindestens einen Wirkstoff umfasst, der unter die Klasse 2, 3 oder 4 des BCS (biopharmazeutischen Klassifizierungssystems) fällt.

3. Selbstemulgierendes festes Partikel nach Anspruch 1 oder 2, wobei das feste Partikel 0,1 bis 0,5 Gew.-%, bezogen auf das Gewicht des Partikels, mindestens eines Antioxidans umfasst, wobei das mindestens eine Antioxidans vorzugsweise im Bereich von 0,2 bis 0,3 Gew.-%, bezogen auf das Gewicht des Partikels, vorliegt.

4. Selbstemulgierendes festes Partikel nach einem der Ansprüche 1 bis 3, wobei die Fettsubstanz mit einem Schmelzpunkt im Bereich von 40 °C bis 90 °C im Bereich von 60 bis 80 Gew.-%, vorzugsweise von 60 bis 70 Gew.-%, bevorzugter von 63 bis 66 Gew.-%, bezogen auf das Gewicht des Partikels, vorliegt.

5. Selbstemulgierendes festes Partikel nach einem der Ansprüche 1 bis 4, wobei die mindestens eine Fettsubstanz mit einem Schmelzpunkt im Bereich von 40 °C bis 90 °C Glycerinbehenat ist.

6. Selbstemulgierendes festes Partikel nach einem der Ansprüche 1 bis 5, wobei die mindestens eine Fettsubstanz mit einem Schmelzpunkt von weniger als 40 °C im Bereich von 10 bis 40 Gew.-%, vorzugsweise von 15 bis 25 Gew.-%, bevorzugter von 18 bis 22 Gew.-%, bezogen auf das Gewicht des festen Partikels, vorliegt.

7. Selbstemulgierendes festes Partikel nach einem der Ansprüche 1 bis 6, wobei die mindestens eine Fettsubstanz mit einem Schmelzpunkt von weniger als 40 °C Kakaobutter ist.

8. Selbstemulgierendes festes Partikel nach einem der Ansprüche 1 bis 7, wobei der mindestens eine Emulgator im Bereich von 10 bis 30 Gew.-%, vorzugsweise von 15 bis 20 Gew.-%, bevorzugter von 15 bis 19 Gew.-%, bezogen auf das Gewicht des Partikels, vorliegt.

9. Selbstemulgierendes festes Partikel nach einem der Ansprüche 1 bis 8, wobei der mindestens eine Emulgator ausgewählt ist aus der Gruppe, bestehend aus Polysorbat 20, Polysorbat 40, Polysorbat 45, Polysorbat 60, Polysorbat 65, Polysorbat 80, wobei der Emulgator vorzugsweise Polysorbat 80 ist.

10. Selbstemulgierendes festes Partikel nach einem der Ansprüche 1 bis 9, wobei das mindestens eine Antioxidans Vitamin E ist, vorzugsweise ist das mindestens eine Antioxidans eine Mischung aus zwei oder mehr von alpha-Tocopherol, beta-Tocopherol, gamma-Tocopherol, delta-Tocopherol.

11. Selbstemulgierendes festes Partikel nach einem der Ansprüche 1 bis 10, das einen mittleren Durchmesser zwischen 125 µm und 250 µm aufweist, wie durch Partikelgrößenanalyse mittels Siebverfahren unter Verwendung eines OCTAGON-200-Vibrationssiebs von ENDECOTTS gemessen.

12. Selbstemulgierendes festes Partikel nach einem der Ansprüche 1 bis 11, wobei das Partikel bei Raumtemperatur fest ist und bei Körpertemperatur, die im Bereich von 36 bis 40 °C liegt, selbstemulgierend ist.

13. Selbstemulgierendes festes Partikel nach einem der Ansprüche 1 bis 12, wobei das feste Partikel bei Vorliegen einer Körpertemperatur, die im Bereich von 36 bis 40 °C liegt, in der Lage ist, sich in Darmflüssigkeit mit neutralem pH selbst zu dispergieren und Mizellen zu erzeugen, die einen hydrodynamischen Durchmesser im Bereich von 100 bis 900 nm aufweisen, wie durch dynamische Lichtstreuung (DLS, dynamic light scattering) gemessen, und bis zu 180 Minuten stabil sind.

14. Mikroverkapselung, die das selbstemulgierende feste Partikel nach den Ansprüchen 1, 3-13 und einen oder mehrere Wirkstoffe umfasst, die unter die Klasse 2, 3 oder 4 des BCS (biopharmazeutischen Klassifizierungssystems) fallen.

15. Selbstemulgierendes festes Partikel nach einem der Ansprüche 1, 3-13 zur Verwendung bei der Abgabe von einem oder mehreren Wirkstoffen, die unter die Klasse 2, 3 oder 4 des BCS (biopharmazeutischen Klassifizierungssystems) fallen.

## Revendications

1. Particule solide auto-émulsifiante à la température corporelle comprenant :
- de 50 % à 90 % en poids, par rapport au poids de la particule, d'au moins une substance grasse ayant un point de fusion compris entre 40 °C et 90 °C,
- de 5 % à 50 % en poids, par rapport au poids de la particule, d'au moins une substance grasse ayant un point de fusion inférieur à 40 °C, et
- de 1 à 50 %, par rapport au poids de la particule, d'au moins un émulsifiant,
dans laquelle
ladite au moins une substance grasse ayant un point de fusion compris entre 40 °C et 90 °C est choisie dans le groupe constitué par le béhénate de glycérol, l'acide stéarique, le stéarate de magnésium, la stéarine végétale, la cire d'abeille, la cire de carnauba et la cire de candelilla,
ladite au moins une substance grasse ayant un point de fusion inférieur à 40 °C est choisie dans le groupe constitué par le beurre de cacao, la margarine, le beurre, l'huile d'olive, l'huile de lin, l'huile de pépins de raisin et les triglycérides à chaînes moyennes (TCM), et le au moins un émulsifiant est un polysorbate (monooléate de polyoxyéthylène sorbitane).

2. Particule solide auto-émulsifiante selon la revendication 1, dans laquelle ladite particule comprend au moins un ingrédient actif relevant de la classe 2, 3 ou 4 du BCS (système de classification biopharmaceutique).

3. Particule solide auto-émulsifiante selon la revendication 1 ou 2, dans laquelle ladite particule solide comprend de 0,1 à 0,5 %, par rapport au poids de la particule, d'au moins un agent antioxydant, de préférence ledit au moins un agent antioxydant est compris entre 0,2 et 0,3 % en poids, par rapport au poids de la particule.

4. Particule solide auto-émulsifiante selon l'une quelconque des revendications 1 à 3, dans laquelle la substance grasse ayant un point de fusion compris entre 40 °C et 90 °C est comprise entre 60 % et 80 %, de préférence entre 60 % et 70 %, plus préférablement entre 63 % et 66 % par rapport au poids de la particule.

5. Particule solide auto-émulsifiante selon l'une quelconque des revendications 1 à 4, dans laquelle ladite au moins une substance grasse ayant un point de fusion compris entre 40 °C et 90 °C est le béhénate de glycérol.

6. Particule solide auto-émulsifiante selon l'une quelconque des revendications 1 à 5, dans laquelle la au moins une substance grasse ayant un point de fusion inférieur à 40 °C est comprise entre 10 % et 40 %, de préférence entre 15 % et 25 %, plus préférablement entre 18 % et 22 % en poids, par rapport au poids de la particule solide.

7. Particule solide auto-émulsifiante selon l'une quelconque des revendications 1 à 6, dans laquelle ladite au moins une substance grasse ayant un point de fusion inférieur à 40 °C est le beurre de cacao.

8. Particule solide auto-émulsifiante selon l'une quelconque des revendications 1 à 7, dans laquelle le au moins un émulsifiant est compris entre 10 et 30 %, de préférence entre 15 et 20 %, plus préférablement entre 15 et 19 %, par rapport au poids de la particule.

9. Particule solide auto-émulsifiante selon les revendications 1 à 8, dans laquelle le au moins un émulsifiant est choisi dans le groupe constitué par le Polysorbate 20, le Polysorbate 40, le Polysorbate 45, le Polysorbate 60, le Polysorbate 65, le Polysorbate 80, de préférence l'émulsifiant est le Polysorbate 80.

10. Particule solide auto-émulsifiante selon l'une quelconque des revendications 1 à 9, dans laquelle le au moins un agent antioxydant est la vitamine E, de préférence le au moins un agent antioxydant est un mélange de deux ou plusieurs parmi l'alpha-tocophérol, le bêta-tocophérol, le gamma-tocophérol, le delta-tocophérol.

11. Particule solide auto-émulsifiante selon l'une quelconque des revendications 1 à 10, ayant un diamètre médian compris entre 125 µm et 250 µm, tel que mesuré par analyse granulométrique par une méthode de tamisage utilisant un tamis vibrant ENDECOTTS - OCTAGON 200.

12. Particule solide auto-émulsifiante selon l'une quelconque des revendications 1 à 11, dans laquelle ladite particule est solide à température ambiante et auto-émulsifiante à une température corporelle comprise entre 36 et 40 °C.

13. Particule solide auto-émulsifiante selon l'une quelconque des revendications 1 à 12, dans laquelle ladite particule solide, en présence d'une température corporelle comprise dans la plage de 36 à 40 °C, est capable de s'auto-disperser dans les fluides intestinaux avec des micelles génératrices de pH neutre ayant un diamètre hydrodynamique compris entre 100 et 900 nm tel que mesuré par diffusion dynamique de lumière (DLS) et étant stable jusqu'à 180 minutes.

14. Microcapsule comprenant la particule solide auto-émulsifiante des revendications 1,3 à 13 et un ou plusieurs ingrédients actifs relevant de la classe 2, 3 ou 4 du BCS (système de classification biopharmaceutique).

15. Particule solide auto-émulsifiante selon l'une quelconque des revendications 1, 3 à 13, destinée à être utilisée dans l'administration d'un ou plusieurs ingrédients actifs relevant de la classe 2, 3 ou 4 du BCS (système de classification biopharmaceutique).
